# EUROPEAN PATENT APPLICATION

(11) **EP 4 248 986 A1**
(43) Date of publication of application: **27.09.2023**
(21) Application number: 21895191.1
(22) Date of filing: 23.11.2021
(51) Int. Cl.: A61K 38/16, A61K 9/00, A61P 31/14, A23L 33/195

(54) **USE OF POLYPEPTIDE HAVING SUPEROXIDE DISMUTASE ACTIVITY AND EXTRACELLULAR VESICLES FOR TREATMENT OR PREVENTION OF RESPIRATORY VIRAL INFECTION**

(30) Priority: 23.11.2020 KR 20200158202
(71) Applicant: Genofocus Co., Ltd., Daejeon 34014 (KR)
(72) Inventor: PAN, Jae Gu, Daejeon 34014 (KR); KIM, Eui Joong, Daejeon 34014 (KR); KIM, Jeong Hyun, Daejeon 34014 (KR)
(74) Representative: HGF
(86) International application number: PCT/KR2021/017304
(87) International publication number: WO 2022/108424

(57) **Abstract**

The present invention relates to use of a polypeptide having superoxide dismutase (SOD) activity and/or extracellular vesicles containing SOD, for the prevention and/or treatment of respiratory viral infection or diseases caused by respiratory viral infection in a subject. The present invention also relates to use of a polypeptide having SOD activity derived from a *Bacillus amyloliquefaciens* strain and/or extracellular vesicles containing SOD derived from a *Bacillus subtillis* strain, for the prevention and/or treatment of respiratory viral infection or diseases caused by respiratory viral infection in a subject.

## Description

### Technical Field

The present invention relates to a use of a polypeptide having superoxide dismutase (hereinafter, simply referred to as "SOD") activity derived from a *Bacillus amyloliquefaciens* strain and/or extracellular vesicles containing SOD (hereinafter, simply referred to as "SOD-EV") derived from a *Bacillus subtilis* strain, for the prevention and/or treatment of respiratory viral infection in a subject.

### Background Art

Respiratory viruses, which are one of the causes of respiratory infection, are known to induce enzymes that produce active oxygen (*e*.*g*., nicotinamide adenine dinucleotide phosphate oxidase (NADPH oxidase), xanthine oxidase, *etc.*)*.*

In addition, coronaviruses, representative respiratory viruses, primarily infect the respiratory tract and gastrointestinal tract in mammals and birds. These coronaviruses are known to be one of the infective agents underlying the common cold, along with rhinoviruses, influenza and respiratory syncytial viruses.

Meanwhile, severe acute respiratory syndrome coronavirus 2 (SARS-CoV-2) is a severe acute respiratory disease coronavirus 2 first known in 2019 and is classified as a positive-sense single-stranded RNA virus. The disease infected by this virus is called coronavirus disease 2019, and is abbreviated to COVID-19. The World Health Organization (WHO) officially declared the Coronavirus pandemic, and in November 2020, the number of infected people in Korea is close to 30,000, and about 55.6 million people around the world are found to have been infected, and the COVID-19 is continuously spreading. When infected with the COVID-19, symptoms such as fever, cough, dyspnea, and pneumonia appear after an incubation period of about 2-14 days.

The COVID-19 is a type of virus that causes respiratory tract infections, and is the virus first discovered in the city of Wuhan, Hubei Province, China in 2019, and belongs to the Coronaviridae group and is known as a virus similar to Severe acute respiratory syndrome coronavirus (SARS-CoV) and Middle East respiratory syndrome coronavirus (MERS-CoV).

Clinical trials for COVID-19 were conducted on the drugs such as Kaletra (main ingredient: lopinavir), a treatment for Human Immunodeficiency Virus (HIV), or remdesivir, a treatment for Ebola virus, and in October 2020, the U.S. FDA approved remdesivir as an indication for the treatment of COVID-19, but this is not a drug with a therapeutic effect that has both the prevention and treatment effects of COVID-19. Therefore, it is important to find a new therapy that can prevent and treat respiratory virus infections, such as coronavirus, including COVID-19.

### Disclosure of Invention

### Technical Problem

An object of the present invention is to provide a method for the prevention and/or treatment of respiratory viral infection or diseases caused by respiratory viral infection, the method including administering a therapeutically effective amount of a polypeptide having superoxide dismutase (SOD) activity and/or extracellular vesicles containing SOD.

Another object of the present invention is to provide a method for the prevention and/or treatment of respiratory viral infection or diseases caused by respiratory viral infection, the method including administering a therapeutically effective amount of a polypeptide having SOD activity derived from a *Bacillus amyloliquefaciens* strain and/or extracellular vesicles containing SOD derived from a *Bacillus subtilis* strain.

Further another object of the present invention is to provide a method for the prevention and/or treatment of coronavirus infection or diseases caused by coronavirus infection, the method including administering a therapeutically effective amount of a polypeptide having SOD activity derived from a *Bacillus amyloliquefaciens* strain and/or extracellular vesicles containing SOD derived from a *Bacillus subtilis* strain.

Still another object of the present invention is to provide a pharmaceutical composition for the prevention and/or treatment of respiratory viral infection, the composition including a polypeptide having SOD activity and/or extracellular vesicles containing SOD.

Yet another object of the present invention is to provide a pharmaceutical composition for the prevention and/or treatment of respiratory viral infection, the composition including a polypeptide having SOD activity derived from a *Bacillus amyloliquefaciens* strain and/or extracellular vesicles containing SOD derived from a *Bacillus subtilis* strain.

Yet still another object of the present invention is to provide a pharmaceutical composition for the prevention and/or treatment of coronavirus infection, the composition including a polypeptide having SOD activity derived from a *Bacillus amyloliquefaciens* strain and/or extracellular vesicles containing SOD derived from a *Bacillus subtilis* strain.

Further another object of the present invention is to provide a food composition for the prevention and/or amelioration of respiratory viral infection, the composition including a polypeptide having SOD activity and/or extracellular vesicles containing SOD.

Still another object of the present invention is to provide a food composition for the prevention and/or amelioration of respiratory viral infection, the composition including a polypeptide having SOD activity derived from a *Bacillus amyloliquefaciens* strain and/or extracellular vesicles containing SOD derived from a *Bacillus subtilis* strain.

Yet another object of the present invention is to provide a food composition for the prevention and/or amelioration of coronavirus infection, the composition including a polypeptide having SOD activity derived from a *Bacillus amyloliquefaciens* strain and/or extracellular vesicles containing SOD derived from a *Bacillus subtilis* strain.

Yet still another object of the present invention is to provide a use of the pharmaceutical composition for the prevention and/or treatment of respiratory viral infection or diseases caused by respiratory viral infection.

Further another object of the present invention is to provide a use of the pharmaceutical composition for preparing a drug for the prevention and/or treatment of respiratory viral infection or diseases caused by respiratory viral infection.

Still another object of the present invention is to provide a use of the food composition for the prevention and/or amelioration of respiratory viral infection or diseases caused by respiratory viral infection.

### Solution to Problem

The inventors of the present invention have confirmed that when the polypeptide having SOD activity derived from a *Bacillus amyloliquefaciens* strain is used in a mammal alone or together with extracellular vesicles containing SOD derived from a *Bacillus subtilis* strain, it exhibits an effect of preventing, treating, or ameliorating coronavirus infection, and completed the present invention.

### Advantageous Effects of Invention

The polypeptide having SOD activity derived from a *Bacillus amyloliquefaciens* strain or the extracellular vesicles containing SOD derived from a *Bacillus subtilis* strain according to the present invention when used alone or used together exhibit an excellent effect of preventing, treating, or ameliorating respiratory viral infection or diseases caused thereby.

### Brief Description of Drawings

FIG. 1 shows particle size analysis results of extracellular vesicles containing SOD derived from a *Bacillus subtilis* strain.
FIG. 2 shows particle size analysis results of extracellular vesicles containing SOD derived from a *Bacillus subtilis* strain.
FIG. 3 shows an animal experimental design for identifying the prophylactic and therapeutic effects of a polypeptide having SOD activity derived from a *Bacillus amyloliquefaciens* strain and/or extracellular vesicles containing SOD derived from a *Bacillus subtilis* strain on coronavirus infection.
FIG. 4 shows the therapeutic effect of a polypeptide having SOD activity derived from a *Bacillus amyloliquefaciens* strain and/or extracellular vesicles containing SOD derived from a *Bacillus subtilis* strain on lung injury caused by coronavirus infection.
FIG. 5 shows the prophylactic effect of a polypeptide having SOD activity derived from a *Bacillus amyloliquefaciens* strain and/or extracellular vesicles containing SOD derived from a *Bacillus subtilis* strain on lung injury caused by coronavirus infection.
FIG. 6 shows that the lung injury observed in Syrian hamsters administered a polypeptide having SOD activity derived from a *Bacillus amyloliquefaciens* strain and/or extracellular vesicles containing SOD derived from a *Bacillus subtilis* strain is significantly different from that in Syrian hamsters not administered the polypeptide and/or the extracellular vesicles containing SOD.
FIG. 7 shows the histopathologic results of observing lungs following the administration of a polypeptide having SOD activity derived from a *Bacillus amyloliquefaciens* strain and/or extracellular vesicles containing SOD derived from a *Bacillus subtilis* strain.
FIG. 8 shows the histopathologic score of lungs according to the therapeutic administration of a polypeptide having SOD activity derived from a *Bacillus amyloliquefaciens* strain and/or extracellular vesicles containing SOD derived from a *Bacillus subtilis* strain.
FIG. 9 shows the histopathologic score of lungs according to the prophylactic administration of a polypeptide having SOD activity derived from a *Bacillus amyloliquefaciens* strain and/or extracellular vesicles containing SOD derived from a *Bacillus subtilis* strain.

### Best Mode for Carrying out the Invention

In one aspect of the present invention, there is provided a method for the prevention and/or treatment of respiratory viral infection or diseases caused by respiratory viral infection, the method including administering a therapeutically effective amount of: (i) a polypeptide having superoxide dismutase (SOD) activity; (ii) extracellular vesicles containing SOD; or (iii) a polypeptide having SOD activity and extracellular vesicles containing SOD.

In this case, the respiratory viral infection may be coronavirus infection. The coronavirus infection is selected from cold, pneumonia, interstitial pneumonia, severe acute respiratory syndrome (SARS), Middle East respiratory syndrome (MERS), sinusitis, COVID-19 infection, otitis, or pharyngitis. In addition, the diseases caused by respiratory viral infection may be viral lung injury or lung disease.

In addition, the coronavirus may be an alphacoronavirus, a betacoronavirus, a gammacoronavirus, or a deltacoronavirus. In particular, the coronavirus may be a human coronavirus.

The polypeptide having SOD activity of the present invention has an amino acid sequence of SEQ ID NO: 1. The polypeptide having SOD activity of the present invention is also interpreted as including an amino acid sequence having substantial identity to the amino acid sequence. The substantial identity means an amino acid sequence having at least 80% homology, more preferably 96% homology, and most preferably 98% homology when the amino acid sequence of the present invention is aligned to correspond to any other sequence as much as possible and the aligned sequence is analyzed using an algorithm commonly used in the art.

The polypeptide may be isolated from a *Bacillus amyloliquefaciens* GF423 strain. The *Bacillus amyloliquefaciens* GF423 strain has been internationally deposited in the Korea Research Institute of Bioscience and Biotechnology on March 6, 2017 under Accession No. KCTC 13222 BP, and the characteristics and culture method of the strain are described in Korean Patent No. 1762199.

The extracellular vesicles containing SOD derived from a *Bacillus subtilis* strain of the present invention means extracellular vesicles having SOD activity. The extracellular vesicles mean a heterogeneous collection of nanosized vesicles surrounded by membranes with complex hydrates including proteins, lipids, and nucleic acids.

The extracellular vesicles containing SOD may be isolated from a *Bacillus subtilis* SOD strain.

In the present invention, the polypeptide having SOD activity derived from a *Bacillus amyloliquefaciens* strain and/or the extracellular vesicles containing SOD derived from a *Bacillus subtilis* strain may be utilized to suppress viral propagation in an individual already infected with respiratory viruses. Preferably, the polypeptide having SOD activity derived from a *Bacillus amyloliquefaciens* strain and/or the extracellular vesicles containing SOD derived from a *Bacillus subtilis* strain may suppress viral propagation in an individual already infected with coronaviruses. More preferably, the polypeptide having SOD activity derived from a *Bacillus amyloliquefaciens* strain and/or the extracellular vesicles containing SOD derived from a *Bacillus subtilis* strain may suppress viral propagation in an individual infected with SARS or COVID-19.

In addition, the polypeptide having SOD activity and the extracellular vesicles containing SOD may be administered simultaneously or sequentially.

In another aspect of the present invention, there is provided a method for the prevention and/or treatment of respiratory viral infection or diseases caused by respiratory viral infection, the method including administering a therapeutically effective amount of a polypeptide having SOD activity and/or extracellular vesicles containing SOD and/or a pharmaceutically acceptable salt thereof. The respiratory viral infection may be coronavirus infection. In addition, the coronavirus infection may be SARS-CoV-2 infection.

In further another aspect of the present invention, there is provided a pharmaceutical composition for the prevention and/or treatment of respiratory viral infection or diseases caused by respiratory viral infection, the composition including a polypeptide having SOD activity and/or extracellular vesicles containing SOD. In this case, the respiratory viral infection may be coronavirus infection.

In yet another aspect of the present invention, there is provided a pharmaceutical composition for the prevention and/or treatment of respiratory viral infection or diseases caused by respiratory viral infection, the composition including a polypeptide having SOD activity derived from a *Bacillus amyloliquefaciens* strain and/or extracellular vesicles containing SOD derived from a *Bacillus subtilis* strain. In this case, the respiratory viral infection may be coronavirus infection.

In still another aspect of the present invention, there is provided a use of the composition for the prevention and/or treatment of respiratory viral infection or diseases caused by respiratory viral infection. In this case, the composition is the same as described above.

In yet still another aspect of the present invention, there is provided a use of the composition for preparing a drug for the prevention and/or treatment of respiratory viral infection or diseases caused by respiratory viral infection. In this case, the composition is the same as described above.

The polypeptide having SOD activity and/or the extracellular vesicles containing SOD of the present invention may be used together with a compound, polypeptide, or the like known to have a prophylactic or therapeutic effect on respiratory viral injection or diseases caused by respiratory viral injection. In addition, the polypeptide having SOD activity derived from a *Bacillus amyloliquefaciens* strain and/or the extracellular vesicles containing SOD derived from a *Bacillus subtilis* strain of the present invention may be used together with a compound, polypeptide, or the like known to have a prophylactic or therapeutic effect on respiratory viral injection or diseases caused by respiratory viral injection.

In further another aspect of the present invention, there is provided a method for the prevention and/or amelioration of respiratory viral infection or diseases caused by respiratory viral infection, the method including administering a sitologically effective amount of a food composition including a polypeptide having SOD activity and/or extracellular vesicles containing SOD.

In still another aspect of the present invention, there is provided a food composition for the prevention or amelioration of diseases caused by respiratory viral infection, the composition including a polypeptide having SOD activity and/or extracellular vesicles containing SOD. More specifically, there is provided a food composition for the prevention or amelioration of diseases caused by respiratory viral infection, the composition including a polypeptide having SOD activity derived from a *Bacillus amyloliquefaciens* strain and/or extracellular vesicles containing SOD derived from a *Bacillus subtilis* strain. In this case, the respiratory viral infection may be coronavirus infection.

In an aspect of the present invention, there is provided a use of the food composition for the prevention and/or amelioration of respiratory viral infection or diseases caused by respiratory viral infection.

The pharmaceutical composition of the present invention may be formulated and used in the form of tablets, pills, powders, granules, capsules, suspensions, solutions, emulsions, syrups, aerosols, injection solutions, or the like, according to a conventional method. For example, carriers, excipients or diluents which can be included in the pharmaceutical composition of the present invention include lactose, dextrose, sucrose, sorbitol, mannitol, xylitol, erythritol, maltitol, starch, gum acacia, alginates, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil. When formulated, the pharmaceutical composition is prepared with generally used diluents or excipients such as fillers, extenders, binders, wetting agents, disintegrating agents, and surfactants.

The pharmaceutical composition of the present invention may be orally administered or parenterally administered according to a desired method, and when parenterally administered, it is preferable to select a nasal spray, a skin external application or an intraperitoneal injection, an intrarectal injection, a subcutaneous injection, an intravenous injection, an intramuscular injection or an intrathoracic injection.

In this case, solid formulations for oral administration include tablets, pills, powders, granules, capsules, and the like, and such solid preparations may be prepared by mixing a complex composition with at least one excipient, for example, starch, calcium carbonate, sucrose, lactose, gelatin, *etc.* In addition to simple excipients, lubricants such as magnesium stearate and talc may also be used. Liquid formulations for oral administration include suspensions, solutions, emulsions, syrups, *etc.,* and in addition to water and liquid paraffin, which are commonly used simple diluents, various excipients such as wetting agents, sweetening agents, fragrances, and preservatives may be used.

Formulations for parenteral administration may include sterilized aqueous solutions, non-aqueous solutions, suspensions, emulsions, lyophilized preparations, suppositories, or the like. As the non-aqueous solutions or the suspensions, propylene glycol, polyethylene glycol, vegetable oil such as olive oil, injectable ester such as ethyl oleate, or the like may be used.

In addition, the pharmaceutical composition according to the present invention may be administered by inhale. While the drug is directly delivered to the lungs, it may not be toxic and may exhibit a longer duration of action even in a small dose. Administration for inhalation may be administration using a pharmaceutical formulation that can be inhaled through the respiratory tract, nasal cavity, or the like, including inhalable particles or droplets containing a drug. For example, either a dry-powder inhaler (DPI) or a pressurized metered dose inhaler (pMDI) may be used for the administration for inhalation, but is not limited thereto. For example, the drug particles are lightly compacted into a frangible matrix which is contained within the delivery device (a dry-powder inhaler). Upon actuation, the delivery device abrades a portion of the drug particles from the matrix, and disperses them into inspiratory breath delivering the drug particles to the respiratory tract. Alternatively, the drug particles may be a free flowing powder contained within a reservoir in the delivery device (a dry-powder inhaler). The reservoir may be an integral chamber within the device, or a capsule, blister, or a similar performed reservoir that is inserted into the device prior to actuation. Upon actuation, the device disperses a portion of the drug particles from the reservoir and disperses them in the inhalation breath delivering the drug particles to the respiratory tract.

In addition, the pharmaceutical composition of the present invention may further include carriers, excipients, or diluents. Lactose, dextrose, sucrose, sorbitol, mannitol, xylitol, erythritol, maltitol, starch, gum acacia, alginates, gelatin, calcium phosphate, calcium silicate cellulose, methyl cellulose, hydroxy propyl methyl cellulose, microcrystalline cellulose, polyvinyl pyrrolidone, water, methyl hydroxybenzoate, propyl hydroxybenzoate, minerals such as talc, magnesium stearate or silicon dioxide, and the like may be used as the carriers, excipients or diluents.

The pharmaceutical composition of the present invention should be administered in a therapeutically effective amount. As used herein, the term "therapeutically effective amount" refers to an amount sufficient to treat diseases at a reasonable benefit/risk ratio applicable to any medical treatment, and the effective dosage level may be determined according to the factors including the patient's body weight, sex, age, condition, the severity of the disease, the activity of drug, the sensitivity to drug, an administration time, an administration route and an excretion rate, duration of treatment, drugs used in combination with the composition, and other factors well known in the medical field. The pharmaceutical composition of the present invention may be administered as an individual therapeutic agent or in combination with other therapeutic agents, and may be administered sequentially or simultaneously with a conventional therapeutic agent. Such administration may be single or multiple administration. It is important to administer an amount capable of obtaining the maximum effect with a minimum amount without adverse effects, and such an amount may be easily determined by a person skilled in the art.

### Mode for Carrying out the Invention

Hereinafter, the present invention will be described in detail according to examples for better understanding of the present invention. However, the following examples are only illustrating the present invention, and the scope of the present invention is not limited thereto.

### Example 1. Isolation/Purification of Superoxide Dismutase (SOD) Polypeptide from Bacillus amyloliquefaciens GF423

### 1.1 Culture of Bacillus amyloliquefaciens GF423

For culturing the *Bacillus amyloliquefaciens* GF423 strain, a single colony formed in LB agar medium (Luia-Bertani (LB) agar; tryptophan 10 g/L, yeast extract 5 g/L, NaCl 10 g/L, agar 15 g/L) was inoculated into 30 mL of LB medium and cultured at 37 °C for 12 hours. This species culture was inoculated again into 3 L of LB medium containing 1 mM manganese sulfate (MnSO₄) and cultured at 37 °C for 20 hours.

### 1.2 Isolation and Purification of Superoxide Dismutase

The cell culture broth was centrifuged at 4 °C at 3,578xg for 20 minutes to obtain the supernatant, and then the supernatant was concentrated 10-fold using Ultrafiltration (hereinafter, UF (MWCO 10,000)). While 300 mL of the concentrated supernatant was stirred at 4 °C, ammonium sulfate was added thereto up to 60% saturation, and the resultant mixture was stirred for 30 minutes. Thereafter, the mixture was centrifuged at 3,578xg for 30 minutes to obtain the supernatant, and the supernatant was loaded onto the HiPrepTM Phenyl HP 16/10 column equilibrated with 50 mM potassium phosphate (pH 7.5) containing 2 M ammonium sulfate. Then, elution was performed using 50 mM potassium phosphate (pH 7.5) containing 2 M to 0.1 M ammonium sulfate.

The SOD-containing fractions (#35-#40) were collected, concentrated using UF (MWCO 10,000), and dialyzed with 50 mM potassium phosphate (pH 7.5) to remove the salt. The concentration of protein was measured by the Bradford assay (Bradford M, Anal Biochem, 72, 248-254, 1976).

The activity of superoxide dismutase was confirmed using a superoxide dismutase assay kit (Cayman Chemical, Michigan, USA). One unit of superoxide dismutase activity is defined as the amount of enzyme that inhibits superoxide radicals by 50%. The activity of the purified SOD was 2231.12±269 U/mg, and the molecular weight on the SDS was about 22,000 dalton.

### Example 2. Isolation/Purification of Extracellular Vesicles Containing SOD from Bacillus subtilis strain

### 2.1. Culture of Bacillus subtilis SOD Strain

The *Bacillus subtilis* SOD strain, which the present inventors had cryopreserved, was used as a strain for the isolation and purification of superoxide dismutase. DSM (Nutrient broth 8 g/L, KCl 1 g/L, MgSO₄·7H₂O 0.12 g/L, 1 mM Ca(NO₃)₂, 0.01 mM MnCl₂·4H₂O, and 0.001 mM FeSO₄) was used as a basal medium. Round-bottom tube and flask culture were performed using a shaking incubator, and the agitation speed was set to 200 rpm and the temperature was set to 37 °C. A 5-L Jar fermenter (Fermentec Co., Ltd., FMT-ST-M05) was used as a fermenter, and the agitation speed was set to 200 rpm, the temperature was set to 37 °C, the aeration rate was set to 1.0 vvm, and the pH was set to 6.8.

A preculture of the *Bacillus subtilis* strain was performed by taking a single colony on the SOD plate, inoculating the colony into a round-bottom tube containing 3 mL of the basal medium, and culturing it for 9 hours. In addition, 3 mL of the culture medium cultured in the round bottom tube was inoculated into a 500-mL flask containing 30 mL of the basal medium, cultured for 16 hours, and then used for main culture inoculation. In the fermenter, the culture was performed with 3.0 L, and the inoculation amount was 1% and the culture was performed for 24 hours.

### 2.2. SOD-EV Isolation

As described above, the *Bacillus subtilis* SOD strain was cultured in a jar fermenter for 24 hours, and then the culture medium was centrifuged at 12,000 rpm at 4 °C for 30 minutes to obtain the culture medium supernatant. In the culture medium supernatant, the remaining bacteria in the culture medium was removed using a 0.22-µm cassette of the TFF system and only the pure culture medium supernatant was separated. The pure culture supernatant was concentrated 200-fold using a 100-kDa cut-off membrane of the TFF system to obtain extracellular vesicles containing SOD (SOD-EV).

### 2.3. Quantification and Identification of SOD-EV

Protein electrophoresis was performed on the SOD-EV obtained by the above-described method, and the protein amount of SOD-EV was measured using Bradford analysis and BCA analysis.

### [Protein electrophoresis]

Sodium dodecyl sulfate-polyacrylamide gel electrophoresis (SDS-PAGE) 4-20% gradient gel (Biorad, Mini-protean TGX precast gels) was used. As the SDS-PAGE buffer solution, 25 mM Tris-base, 192 mM glycine, and 0.1% SDS (pH 8.3) were used, and the SOD-EV was mixed with a 5x sample buffer and used after heat treatment at 100 °C for 10 minutes. After protein electrophoresis, proteins were identified by Coomassie staining and western blot. After staining the protein with Coomassie brilliant blue for 30 minutes, the reaction was conducted with a destaining buffer for 3 hours.

### [Western blot]

After the SDS-PAGE of the SOD-EV, the proteins were transferred to a 0.22-µm PVDF membrane, and the membrane was then blocked in 5% skim milk for 1 hour. Then, it was washed three times with a washing buffer, an anti-SOD antibody (1:10,000 skim milk) was then attached for 3 hours at at room temperature. Thereafter, it was washed three times using a washing buffer, secondary antibodies (1:100,000 skim milk) were attached thereto, and the reaction was performed at room temperature for 1 hour. Then, it was washed three times using a washing buffer and confirmed through ECL reaction.

Mini-Protean II (Bio-Rad, Inc.) was used as a protein electrophoresis device, and Chemi-doc MP imaging system (Bio-Rad, Inc.) was used as a confirmation device.

### [Determination of Size of Extracellular Vesicles Containing SOD (SOD-EV)]

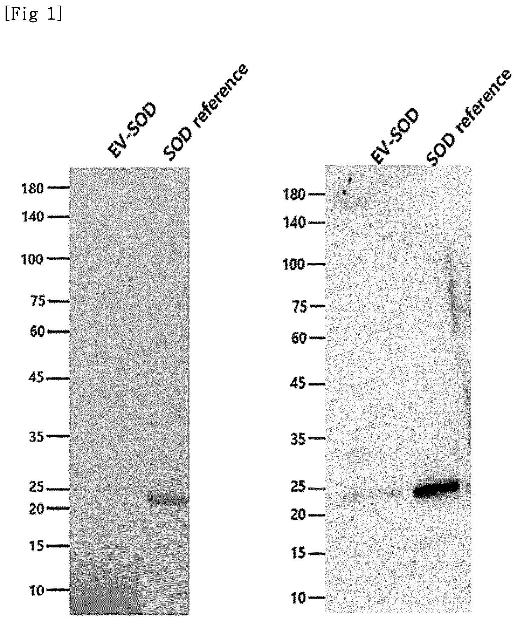

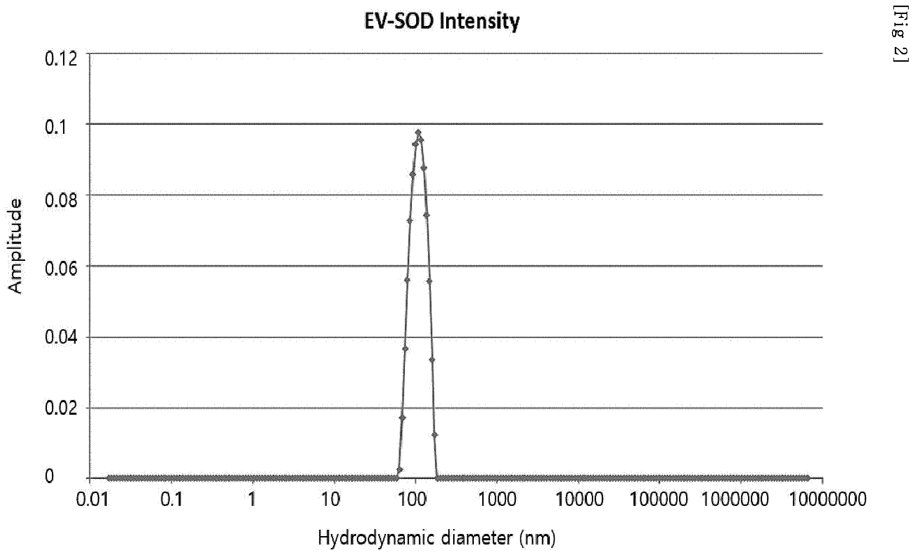

Dynamic light scattering (DLS) was used to measure the size of the purified SOD-EV. SOD-EV samples were placed in wells in 8 µL each and placed in a chamber of the DLS device, and the particle size was measured using laser light. FIG. 1 and FIG. 2 show particle size analysis results. The protein concentration of the SOD-EV was 0.5 mg/mL in the Bradford analysis and 0.124 mg/mL in the BCA analysis (see Table 1 below).

**[Table 1]**

| | SOD-EV concentration (mg/mL) |
|---|---|
| Bradford analysis | 0.5 |
| BCA analysis | 0.124 |

### Example 3. Prophylactic and Therapeutic Effects of SOD and SOD-EV on Coronavirus Infection

### Example 3-1. Visual Evaluation of Lungs Following Prophylactic and Therapeutic Administration of SOD and SOD-EV

Forty (40) 6-week-old Syrian hamsters (Male) were purchased from Central Lab. Animal Inc. for animal experiments to confirm the prophylactic and therapeutic effects of SOD and SOD-EV on coronavirus infection. The purchased Syrian hamsters were divided into eight (8) groups as shown in Table 2 below, and SOD, SOD-EV, and SOD together with SOD-EV were respectively administered to the hamsters, and Sars-Cov2 as a respiratory virus was used as a challenge virus.

**[Table 2]**

| Gro up | Size | Group description | Sars-Cov2 challenge (500 PFU) | TI path | TI & Dosage | Regimen |
|---|---|---|---|---|---|---|
| G1 | N=5 | Therapeutic mode | Yes | Nasal spray | SOD-EV 600 ng | Day 0 to 4, once daily |
| G2 | N=5 | | | Nasal spray | SOD 1 U | Day 0 to 4, once daily |
| G3 | N=5 | | | Nasal spray | SOD-EV 600 ng + SOD 1 U | Day 0 to 4, once daily |
| G4 | N=5 | Preventive mode | Yes | Nasal spray | EV 600 ng | Day -2 to 4, once daily |
| G5 | N=5 | | | Nasal spray | SOD 1 U | Day -2 to 4, once daily |
| G6 | N=5 | | | Nasal spray | SOD-EV 600 ng + SOD 1U | Day -2 to 4, once daily |
| G7 | N=5 | Use only virus | Yes | Nasal spray | N/A | - |
| G8 | N=5 | No challenge | N/A | Nasal spray | SOD-EV 600 ng + SOD 1 U | Day -2 to 4, once daily |

As a group for testing the treatment effect of coronavirus infection, G1 to G3 were classified as treatment modes.SOD-EV, SOD, and SOD-EV with SOD were administered to G1, G2, and G3, respectively, daily from the day of Sars-Cov2 challenge to 4 days after the challenge.

As a group for testing the effect of preventing coronavirus infection, G4 to G6 were classified as prevention mode. SOD-EV, SOD, and SOD-EV with SOD were administered to G4, G5, and G6, respectively, once daily from 2 days before Sars-Cov2 challenge to 4 days after the challenge.

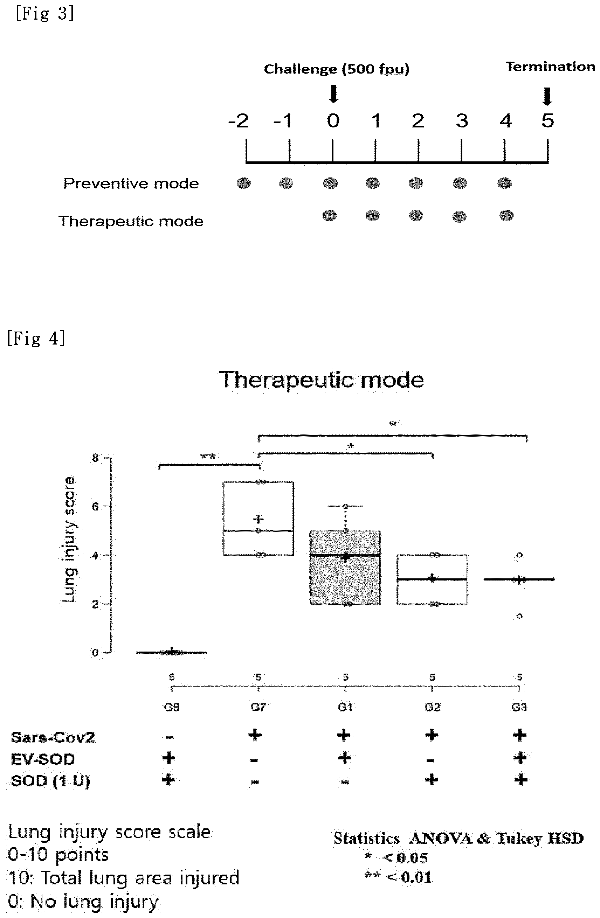

G7 was challenged with the virus without any test material, and G8 was administered only SOD-EV and SOD without virus challenge. Experimental designs for therapeutic mode and preventive mode each are as shown in FIG. 3.

Except for G8 which was not challenged with Sars-Cov2, lung injuries were observed after the Sars-Cov2 challenge in all of G1 to G7.

However, the lung injuries scores of G1 to G3, where SOD and/or SOD-EV was administered after the Sars-Cov2 challenge, were less than that of G7 (see FIG. 4). In particular, the lung injuries shown in G2 administered only SOD and G3 administered SOD-EV together with SOD showed a statistically significant difference compared to the lung injury observed in G7 (p<0.05), which indicates that when SOD alone or SOD and EV were used together, the treatment effect of an infection caused by respiratory viruses such as Sars-Cov2 was excellent.

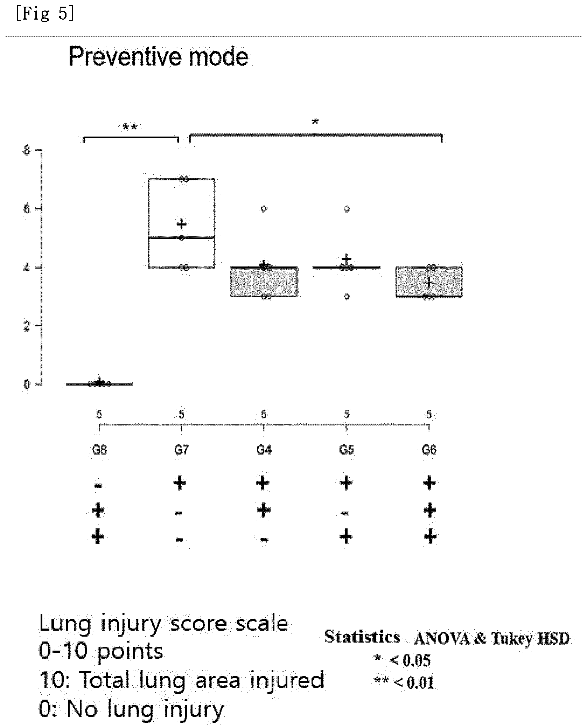

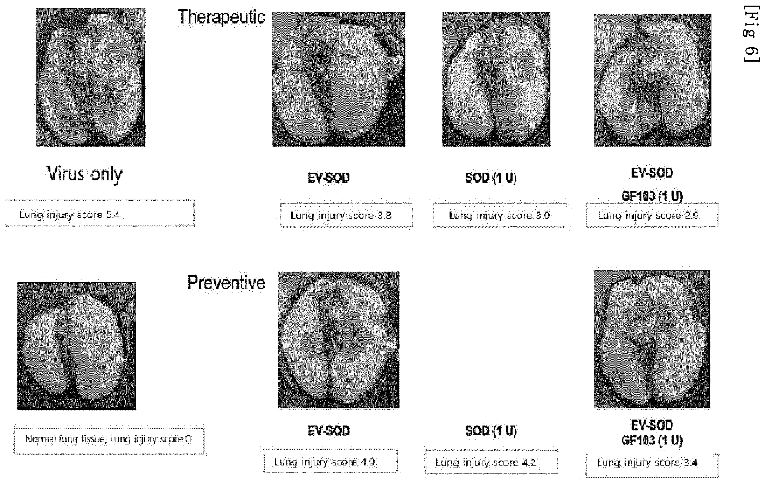

In addition, G4, G5 and G6 which were administered SOD and/or SOD-EV 2 days before challenge, showed less lung injuries than G7 which was not administered SOD and/or SOD-EV (see FIGS. 5 and 6). In particular, G6, which was administered SOD-EV and SOD together, showed a statistically significant difference in lung injury compared to G7 (p<0.05). These experimental results show that when SOD and EV are used together, the effect of preventing infection caused by respiratory viruses such as Sars-Cov2 is excellent.

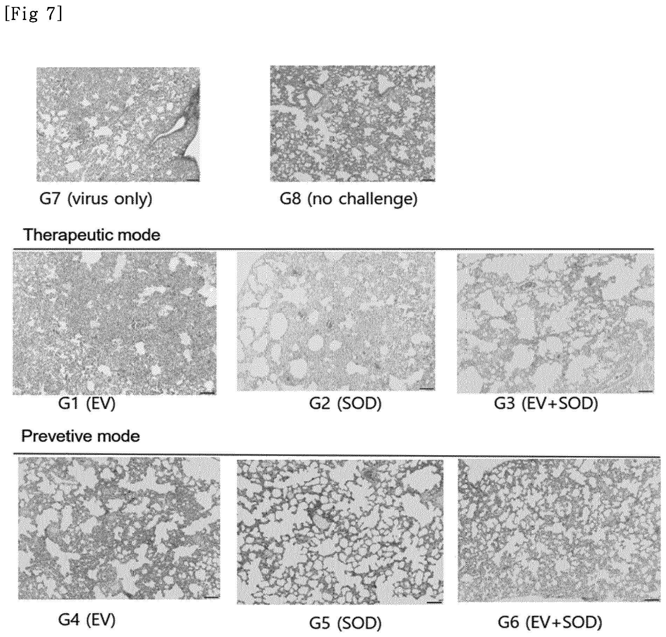

In addition, there was less pulmonary edema and inflammation in G1 to G3 administered SOD-EV and/or SOD than those identified in G7 (see Therapeutic mode in FIG. 7).

Meanwhile, there was less pulmonary edema and inflammation in G4 to G6 administered SOD-EV and/or SOD before the Sars-Cov2 challenge than in G7 (see Preventive mode in FIG. 7).

### Example 3-2. Histopathologic Evaluation of Lungs Following Prophylactic and Therapeutic Administration of SOD and SOD-EV

In order to confirm the prophylactic and therapeutic effects of SOD and SOD-EV on coronavirus infection, histopathologic examination of lungs in the eight (8) groups evaluated with the naked eye in Example 3-1 was performed.

Specifically, tissue slices were obtained from the lungs in the eight (8) groups, and then each tissue slice was fixed in a 10% neutral buffered formalin solution for 24 hours. The fixed tissue slice was put into an automatic tissue processor to remove moisture in the tissue, and paraffin was infiltrated into the tissue. After the infiltration process was completed, the tissue slice was put into a frame, paraffin was poured into the frame to form a block having a certain shape, and the formed block was cooled and then cut into a thickness of 4 µm using a microtome.

H&E staining was performed on the slides produced through the above process as follows.

First, slides with paraffin tissue slices were put into different xylenes, respectively, for 5 minutes and deparaffinized, and then rehydrated by treatment with 100% ethanol twice for 3 minutes, 80% ethanol for 2 minutes, and 70% ethanol for 2 minutes.

After the slices were washed with tap water for 5 minutes, the tissue slides were placed in Harris hematoxylin for 10 minutes and washed with tap water for 3 minutes. The slices were quickly immersed twice in 0.5% acidic alcohol to remove residues, washed with tap water for 1 minute, and the washed slices were put in a 1% ammonia solution for 2 minutes to show blue. Thereafter, the slices were slightly immersed in 95% ethanol for 10 seconds and then stained with eosin for 1 minute and 30 seconds. The stained slices were dehydrated three times for 50 seconds in 100% ethanol and put in xylene to clear twice for 5 minutes.

After dropping one drop of the aqueous fixing solution on the coverslip of the slides subjected to H&E staining, the slides were dried and the results were observed through an optical microscope.

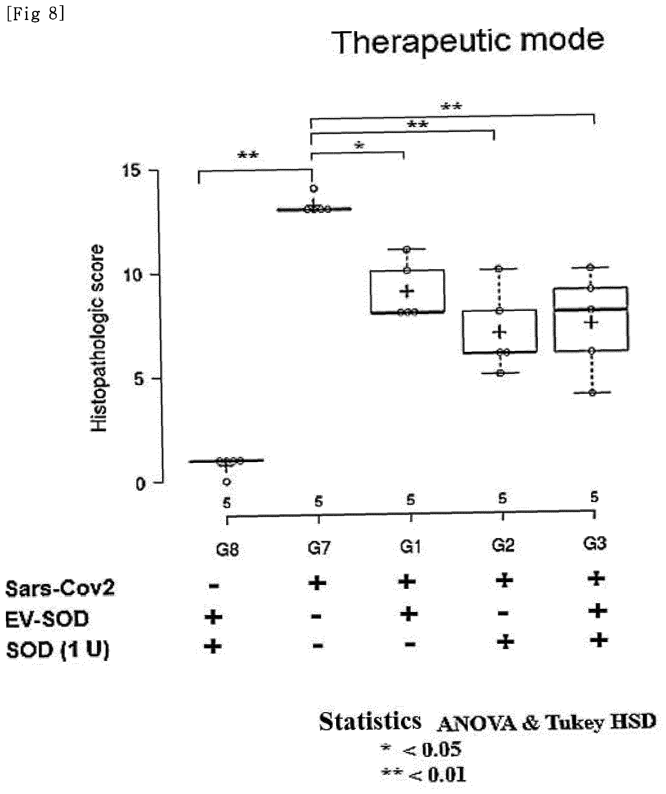

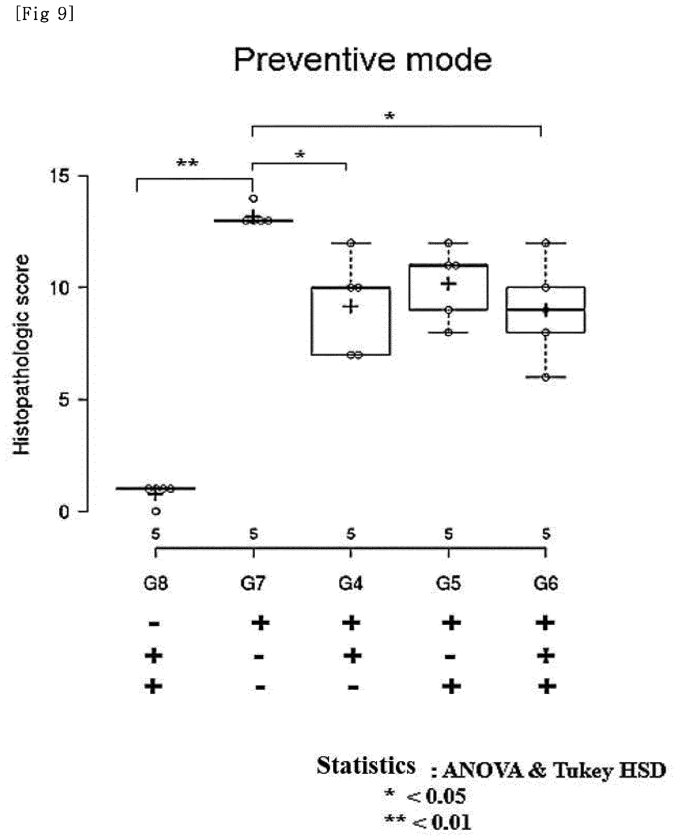

Histopathologic examination was performed using Table 3 as a measure of histopathologic scores, and the results are shown in Table 4, and FIGS. 8 and 9 below.

**[Table 3]**

| **Histopathologic criteria** | **Value** | | | |
|---|---|---|---|---|
| | **0** | **1** | **2** | **3** |
| **Hemorrhage** | 0%-10% of hemorrhage (exclusive of 10%) | Slight hemorrhage (10%-30% of area) | Moderate hemorrhage (30%-60% of area) | Severe hemorrhage (greater than 60% of area) |
| **Inflammatory cell infiltrates** | 0%-10% of leukocyte infiltrates (exclusive of 10%) | Slight leukocyte infiltrates (10%-30% of area) | Moderate leukocyte infiltrates (30%-60% of area) | Severe leukocyte infiltrates (greater than 60% of area) |
| **Necrotic/apopto tic bodies** | 0%-10% of necrotic/apopt otic bodies in parenchyma (exclusive of 10%) | Slight necrotic/apopt otic bodies in parenchyma (10%-30% of area) | Moderate necrotic/apopt otic bodies in parenchyma (30%-60% of area) | Severe necrotic/apopt otic bodies in parenchyma (greater than 60% of area) |
| **Alveolar macrophage aggregate** | 0%-10% of alveolar macrophage aggregate (exclusive of 10%) | Slight alveolar macrophage aggregate (10%-30% of area) | Moderate alveolar macrophage aggregate (30%-60% of area) | Severe alveolar macrophage aggregate (greater than 60% of area) |
| **Consolidation** | 0%-10% of inflammatory exudate in bronchi/alveoli (exclusive of 10%) | Slight inflammatory exudate in bronchi/alveoli (10%-30% of area) | Moderate inflammatory exudate in bronchi/alveoli (30%-60% of area) | Severe inflammatory exudate in bronchi/alveoli (greater than 60% of area) |
| **Pulmonary emphysema** | N/A | Slight pulmonary emphysema (less than 30% of area) | Moderate pulmonary emphysema (30%-60% of area) | Severe pulmonary emphysema (greater than 60% of area) |
| **Bronchial epithelial atrophy** | Normal bronchial epithelium | Slight bronchial epithelial atrophy (less than 30% of area) | Moderate bronchial epithelial atrophy (30%-60% of area) | Severe bronchial epithelial atrophy (greater than 60% of area) |
| **Cell residues in bronchi** | 0%-10% of cell residues present in bronchi (exclusive of 10%) | Slight cell residues present in bronchi (10%-30% of area) | Moderate cell residues present in bronchi (30%-60% of area) | Severe cell residues present in bronchi (greater than 60% of area) |
| **Peri-epithelial monocyte lining** | 0%-10% of peri-epithelial monocyte lining (exclusive of 10%) | Slight peri-epithelial monocyte lining (10%-30% of area) | Moderate peri-epithelial monocyte lining (30%-60% of area) | Severe peri-epithelial monocyte lining (greater than 60% of area) |

**[Table 4]**

| **Group** | | | **No** | **Hem orrh age** | **Infla mm ator y cell infilt rates** | **Necro tic/ap optoti c bodie s** | **Alve olar mac roph age aggr egat e** | **Con solid atio n** | **Pul mon ary emp hyse ma** | **Bro nchi al epit helia l atro phy** | **Cell residu es in bronc hi** | **Peri-epitheli al monoc yte lining** | **Histopat hologic score** |
|---|---|---|---|---|---|---|---|---|---|---|---|---|---|
| The rap euti c mo de | SOD-EV | G1 | #1 | 1 | 2 | 1 | 2 | 1 | 2 | 0 | 1 | 1 | 11 |
| | | G1 | #2 | 1 | 1 | 1 | 1 | 0 | 2 | 0 | 1 | 1 | 8 |
| | | G1 | #3 | 0 | 2 | 2 | 1 | 1 | 1 | 0 | 2 | 1 | 10 |
| | | G1 | #4 | 1 | 1 | 1 | 1 | 1 | 0 | 0 | 1 | 2 | 8 |
| | | G1 | #5 | 1 | 2 | 1 | 1 | 0 | 1 | 0 | 1 | 1 | 8 |
| | | **Average** | | **0.8** | **1.6** | **1.2** | **1.2** | **0.6** | **1.2** | **0** | **1.2** | **1.2** | **9** |
| | Free SOD | G2 | #1 | 1 | 2 | 1 | 2 | 1 | 1 | 0 | 1 | 1 | 10 |
| | | G2 | #2 | 1 | 2 | 1 | 0 | 0 | 0 | 0 | 1 | 1 | 6 |
| | | G2 | #3 | 1 | 2 | 1 | 2 | 0 | 1 | 0 | 1 | 0 | 8 |
| | | G2 | #4 | 0 | 1 | 1 | 0 | 1 | 1 | 0 | 1 | 0 | 5 |
| | | G2 | #5 | 1 | 1 | 1 | 0 | 0 | 1 | 0 | 2 | 0 | 6 |
| | | **Average** | | **0.8** | **1.6** | **1** | **0.8** | **0.4** | **0.8** | **0** | **1.2** | **0.4** | **7** |
| | SOD- | G3 | #1 | 1 | 1 | 1 | 0 | 1 | 0 | 0 | 0 | 0 | 4 |
| | EV + Free SOD | G3 | #2 | 1 | 2 | 2 | 1 | 1 | 1 | 0 | 1 | 1 | 10 |
| | | G3 | #3 | 1 | 1 | 0 | 1 | 0 | 1 | 2 | 0 | 0 | 6 |
| | | G3 | #4 | 2 | 2 | 1 | 2 | 0 | 1 | 0 | 0 | 1 | 9 |
| | | G3 | #5 | 0 | 2 | 1 | 2 | 0 | 1 | 0 | 1 | 1 | 8 |
| | | **Average** | | **1** | **1.6** | **1** | **1.2** | **0.4** | **0.8** | **0.4** | **0.4** | **0.6** | **7.4** |
| Pre ven tive mo de | SOD-EV | G4 | #1 | 1 | 2 | 1 | 1 | 0 | 0 | 0 | 1 | 1 | 7 |
| | | G4 | #2 | 1 | 2 | 1 | 0 | 1 | 1 | 2 | 1 | 1 | 10 |
| | | G4 | #3 | 1 | 2 | 1 | 1 | 1 | 0 | 0 | 0 | 1 | 7 |
| | | G4 | #4 | 1 | 2 | 2 | 2 | 0 | 2 | 3 | 0 | 0 | 12 |
| | | G4 | #5 | 1 | 1 | 2 | 1 | 1 | 1 | 3 | 0 | 0 | 10 |
| | | **Average** | | **1** | **1.8** | **1.4** | **1** | **0.6** | **0.8** | **1.6** | **0.4** | **0.6** | **9.2** |
| | Free SOD | G5 | #1 | 1 | 3 | 2 | 1 | 1 | 1 | 0 | 1 | 1 | 11 |
| | | G5 | #2 | 1 | 2 | 2 | 1 | 0 | 1 | 0 | 1 | 1 | 9 |
| | | G5 | #3 | 2 | 2 | 1 | 2 | 1 | 2 | 0 | 0 | 1 | 11 |
| | | G5 | #4 | 2 | 3 | 1 | 2 | 1 | 1 | 0 | 1 | 1 | 12 |
| | | G5 | #5 | 2 | 2 | 2 | 0 | 0 | 0 | 0 | 1 | 1 | 8 |
| | | **Average** | | **1.6** | **2.4** | **1.6** | **1.2** | **0.6** | **1** | **0** | **0.8** | **1** | **10.2** |
| | SOD-EV + Free SOD | G6 | #1 | 1 | 2 | 2 | 1 | 1 | 1 | 0 | 1 | 1 | 10 |
| | | G6 | #2 | 0 | 1 | 1 | 0 | 0 | 2 | 0 | 1 | 1 | 6 |
| | | G6 | #3 | 1 | 2 | 1 | 0 | 1 | 1 | 0 | 1 | 1 | 8 |
| | | G6 | #4 | 1 | 2 | 2 | 2 | 2 | 0 | 0 | 1 | 2 | 12 |
| | | G6 | #5 | 1 | 2 | 1 | 2 | 1 | 0 | 0 | 1 | 1 | 9 |
| | | **Average** | | **0.8** | **1.8** | **1.4** | **1** | **1** | **0.8** | **0** | **1** | **1.2** | **9** |
| Vir us alo ne | Virus 500PF U /head | G7 | #1 | 1 | 2 | 2 | 1 | 1 | 1 | 2 | 2 | 1 | 13 |
| | | G7 | #2 | 0 | 2 | 1 | 2 | 2 | 1 | 3 | 1 | 1 | 13 |
| | | G7 | #3 | 1 | 3 | 2 | 2 | 1 | 0 | 3 | 1 | 1 | 14 |
| | | G7 | #4 | 1 | 2 | 1 | 2 | 3 | 1 | 2 | 1 | 0 | 13 |
| | | G7 | #5 | 1 | 2 | 2 | 1 | 2 | 1 | 3 | 1 | 0 | 13 |
| | | **Average** | | **0.8** | **2.2** | **1.6** | **1.6** | **1.8** | **0.8** | **2.6** | **1.2** | **0.6** | **13.2** |
| Dru g alo ne | SOD-EV + free SOD No challen ge | G8 | #1 | 0 | 1 | 0 | 0 | 0 | 0 | 0 | 0 | 0 | 1 |
| | | G8 | #2 | 0 | 1 | 0 | 0 | 0 | 0 | 0 | 0 | 0 | 1 |
| | | G8 | #3 | 0 | 0 | 0 | 0 | 0 | 0 | 0 | 0 | 0 | 0 |
| | | G8 | #4 | 0 | 1 | 0 | 0 | 0 | 0 | 0 | 0 | 0 | 1 |
| | | G8 | #5 | 0 | 1 | 0 | 0 | 0 | 0 | 0 | 0 | 0 | 1 |
| | | **Average** | | **0** | **0.8** | **0** | **0** | **0** | **0** | **0** | **0** | **0** | **0.8** |

The histopathologic examination showed that the consolidation and bronchial epithelial atrophy observed in G1 to G6 administered SOD and/or SOD-EV were significantly less than those observed in G7 without the administration of SOD and/or SOD-EV (see Table 4).

In addition, G1, G2, and G3 that were administered SOD and/or SOD-EV after the challenge had lower histopathologic scores than G7 without the administration of SOD and/or SOD-EV (see FIG. 8). In particular, the histopathologic scores shown in G2 administered with only SOD and G3 administered with SOD-EV together with SOD showed a significant difference compared to those shown in G7 (p<0.01).

In addition, G7, which was only challenged with virus, had severe bronchial epithelial atrophy, and thus bronchial epithelium was hardly observed, and G4, G5 and G6, which were administered SOD and/or SOD-EV two days before the challenge, had lower histopathological scores than G7 (see FIG. 9). In particular, the histopathologic scores shown in G6 administered SOD-EV together with SOD showed a significant difference compared to those shown in G7 (p<0.05).

That is, the histopathologic scores showed a consistent pattern to the lung damage score observed with the naked eye, and in particular, it was found that the effects of preventing and treating the consolidation and bronchial epithelial atrophy were excellent.

## Claims

1. A method for the prevention and/or treatment of respiratory viral infection or diseases caused by respiratory viral infection, the method comprising administering a therapeutically effective amount of
(i) a polypeptide having superoxide dismutase (SOD) activity;
(ii) extracellular vesicles containing SOD; or
(iii) a polypeptide having SOD activity and extracellular vesicles containing SOD.

2. The method of claim 1, wherein the diseases caused by respiratory viral infection is selected from the group consisting of cold, pneumonia, interstitial pneumonia, severe acute respiratory syndrome (SARS), Middle East respiratory syndrome (MERS), sinusitis, Sars-Cov2 infection, otitis, and pharyngitis.

3. The method of claim 1, wherein the respiratory virus is a coronavirus.

4. The method of claim 1, wherein the diseases caused by respiratory viral infection are viral lung injury or lung disease.

5. The method of claim 1, wherein the polypeptide having SOD activity and/or the extracellular vesicles containing SOD are administered by a nasal spray.

6. The method of claim 1, wherein the polypeptide having SOD activity and the extracellular vesicles containing SOD are administered simultaneously or sequentially.

7. The method of claim 1, wherein the polypeptide is derived from a *Bacillus amyloliquefaciens* strain.

8. The method of claim 1, wherein the extracellular vesicles containing SOD is derived from a *Bacillus subtilis* strain.

9. A pharmaceutical composition for the prevention and/or treatment of respiratory viral infection or diseases caused by respiratory viral infection, the composition comprising as an active ingredient:
(i) a polypeptide having superoxide dismutase (SOD) activity;
(ii) extracellular vesicles containing SOD; or
(iii) a polypeptide having SOD activity and extracellular vesicles containing SOD.

10. A food composition for the prevention and/or amelioration of respiratory viral infection or diseases caused by respiratory viral infection, the composition comprising as an active ingredient:
(i) a polypeptide having superoxide dismutase (SOD) activity;
(ii) extracellular vesicles containing SOD; or
(iii) a polypeptide having SOD activity and extracellular vesicles containing SOD.

11. A use of the pharmaceutical composition of claim 9 for the prevention and/or treatment of respiratory viral infection or diseases caused by respiratory viral infection.

12. A use of the pharmaceutical composition of claim 9 for preparing a drug for the prevention and/or treatment of respiratory viral infection or diseases caused by respiratory viral infection.

13. A use of the food composition of claim 10 for the prevention and/or amelioration of respiratory viral infection or diseases caused by respiratory viral infection.
